# EUROPÄISCHE PATENTSCHRIFT

(11) **EP 3 781 088 B1**
(45) Veröffentlichungstag und Bekanntmachung des Hinweises auf die Patenterteilung: **20.12.2023**
(21) Anmeldenummer: 19723317.4
(22) Anmeldetag: 16.04.2019
(51) Int. Cl.: A61F 2/95

(54) **VORRICHTUNG ZUR EINBRINGUNG VON IMPLANTATEN**
DEVICE FOR INTRODUCING IMPLANTS
DISPOSITIF D'INTRODUCTION D'IMPLANTS

(30) Priorität: 20.04.2018 DE 102018109580
(43) Veröffentlichungstag der Anmeldung: 24.02.2021
(73) Patentinhaber: Phenox GmbH, 44801 Bochum (DE)
(72) Erfinder: HENKES, Hans, 70192 Stuttgart (DE); HANNES, Ralf, 44137 Dortmund (DE); MONSTADT, Hermann, 44797 Bochum (DE)
(74) Vertreter: Schneiders & Behrendt Bochum
(86) Internationale Anmeldenummer: PCT/EP2019/059783
(87) Internationale Veröffentlichungsnummer: WO 2019/201905

(56) Entgegenhaltungen:
- WO-A1-2005/094727
- US-A1- 2002 151 967
- US-A1- 2008 027 528
- US-A1- 2010 331 948
- US-A1- 2012 265 293

## Beschreibung

Die Erfindung betrifft ein System aus einem endovaskulären, zumindest teilweise zylindrisch aufgebauten Implantat und einer Vorrichtung zur Einbringung des Implantats in Blutgefäße, wobei das Implantat in einem expandierten Zustand, in dem es im Blutgefäß platziert wird, und in einem komprimierten Zustand vorliegt, in dem es in das Blutgefäß einführbar ist, wobei die Vorrichtung einen länglichen Schaft aufweist, auf dessen Außenseite das Implantat im komprimierten Zustand ablösbar festgelegt ist und der durch das Implantat hindurch verläuft.

Die Einbringung von endovaskulären Implantaten in Blutgefäße wie Arterien, Venen, Aneurysmen oder sonstigen vaskulären Fehlbildungen ist bekannter Stand der Technik. Hierbei werden z. B. Stents in einem Blutgefäß implantiert, um dieses dauerhaft offenzuhalten. Weitere Beispiele sind Okklusionswendeln, die in ein Aneurysma eingebracht werden, um dieses auszufüllen und eine Thromboisierung zu induzieren, oder Flow Diverter, welche vor einem Aneurysma platziert werden, um dieses vom Blutfluss abzukoppeln. Typischerweise werden derartige Implantate mittels eines Katheters, welcher an einer geeigneten Stelle in das Blutgefäßsystem eingeführt wird, an die gewünschte Position gebracht und dort platziert. Das Implantat kann z. B. am distalen Ende eines Führungsdrahts angebracht sein, wobei sich zwischen Führungsdraht und Implantat eine Ablösestelle befindet, die durch Anlegen einer elektrischen Spannung korrodiert und sich jedenfalls so weitgehend auflöst, dass eine Ablösung und Freisetzung des Implantats erfolgt. Andere Ablösesysteme beruhen darauf, dass ein mechanischer Formschluss zwischen Implantat und der Vorrichtung zur Einbringung des Implantats aufgehoben wird.

Gerade bei Stents, Flow Divertern und ähnlich aufgebauten Implantaten, die eine innen hohle Zylinderstruktur mit einer Vielzahl von Öffnungen auf der Mantelfläche aufweisen, kann sich der Vorschub des Implantats durch einen Katheter oder ein Blutgefäß schwierig gestalten. Insbesondere muss verhindert werden, dass das Implantat beim Vorschub umknickt oder eine Stauchung erfährt. Entsprechend kann eine Fixierung des Implantats vor der Freisetzung am proximalen und am distalen Ende sinnvoll sein. Gleichwohl muss jedoch eine möglichst sichere, einfache und schnelle Ablösung des Implantats an der Zielposition gewährleistet sein. Es stellt sich somit die Aufgabe, die aus dem Stand der Technik bekannten Ablösesysteme für Implantate weiter zu verbessern.

Ein weiteres Problem, dass sich bei Implantaten aus dem Stand der Technik ergeben kann, ist die Tatsache, dass das letztlich im Blutgefäß verbleibende Implantat nach wie vor Verbindungsstellen zur Einführvorrichtung aufweist. Im Falle einer mechanischen Ablösung können beispielsweise für den Formschluss verantwortliche, am Implantat befindliche Verbindungselemente am Implantat verbleiben. Im Falle der Einführung des Implantats mithilfe eines elektrolytisch ablösbaren Führungsdrahts läuft das Implantat in der Regel in Richtung des proximal angeordneten Führungsdrahts eng zusammen. Mit anderen Worten verbleibt nach der elektrolytischen Ablösung am Implantat ein für die Wirkung des Implantats nicht erforderlicher proximaler Teil. Sowohl ein solcher proximaler Teil als auch die zuvor genannten Verbindungselemente können sich nach Platzierung des Implantats nachteilig auswirken, indem sie beispielsweise in das Lumen des Blutgefäßes hineinragen und den Blutfluss behindern, die Wandung des Blutgefäßes verletzen oder sonstige Probleme verursachen.

Des Weiteren ist bei der herkömmlichen Einbringung von Implantaten, bei der das Implantat durch einen Mikrokatheter vorgeschoben wird, die Größe des komprimierten Implantates naturgemäß begrenzt, da nur ein gewisser Bauraum zur Verfügung steht und der Durchmesser des Implantates im komprimierten Zustand einen bestimmten Wert im Verhältnis zum Innendurchmesser des Mikrokatheters nicht übersteigen kann. Insbesondere im neurovaskulären Bereich werden Mikrokatheter nur bis zu einer bestimmten Größe verwendet. Eine Vorrichtung zur Einbringung von Implantaten, die nicht darauf angewiesen ist, das Implantat durch einen Mikrokatheter zu schieben, sondern auch die Einbringung ohne Mikrokatheter erlaubt, würde somit bei der Gestaltung des Implantats zusätzliche Freiräume eröffnen. Beispielsweise könnte das Implantat größer oder massiver ausgebildet werden oder auch mehr oder dickere Streben aufweisen.

Ein System zur Einführung eines Stents, bei dem der Stent proximal und distal über Verbindungsstücke zu einem Einführsystem verfügt, die sich elektrolytisch lösen lassen, ist der US 2012/0265293 A1 zu entnehmen. Der Stent wird jedoch in einem Katheter an den Zielort gebracht; darüber hinaus verbleiben Verbindungsstücke am freigesetzten Stent.

Erfindungsgemäß stellte sich somit die Aufgabe, ein System aus einem Implantat und einer Vorrichtung zur Einbringung des Implantats in Blutgefäße zur Verfügung zu stellen, die die vorgenannten Probleme überwindet.

Diese Aufgabe wird erfindungsgemäß gelöst durch ein System nach Anspruch 1.

Die erfindungsgemäße Vorrichtung zeichnet sich durch einen länglichen Schaft aus, auf dessen Außenseite das Implantat im komprimierten Zustand gehalten wird. Hierzu weist das Implantat proximal und distal jeweils mindestens eine Verbindungsstelle mit dem Schaft auf, die sich zwecks Freisetzung des Implantats lösen lässt. Die Vorrichtung samt länglichem Schaft und hierauf angebrachtem Implantat kann somit an die gewünschte Position vorgeschoben werden. Bevorzugt erfolgt der Vorschub distal ohne Katheter, d. h. der Schaft wird zwar in der Regel zunächst durch einen verhältnismäßig großlumigen Führungskatheter vorgeschoben, auf den Einsatz eines kleinlumigen Mikrokatheters für den Vorschub in weiter distal gelegene Regionen wird hingegen verzichtet. Grundsätzlich ist aber auch die Verwendung eines Katheters für den gesamten Vorschub denkbar, in diesem Fall wird zumindest der das Implantat tragende Teil des Schafts aus dem Katheter herausgeschoben bzw. der Katheter wird so weit zurückgezogen, dass das Implantat frei liegt.

Wegen der Verbindungsstellen zwischen Implantat und Schaft erfolgt zunächst keine Ablösung des Implantats. Vielmehr kann das Implantat durch Bewegen des Schafts in seiner Position beliebig verändert werden, wenn der behandelnde Arzt erkennt, dass eine Neupositionierung notwendig ist. Bei Bedarf kann der Schaft mit dem Implantat auch nach proximal zurückgezogen, grundlegend neu positioniert oder aus dem Blutgefäßsystem entfernt werden.

Wenn sich der behandelnde Arzt zur Ablösung des Implantats entschieden hat, werden die Verbindungsstellen zwischen Schaft und Implantat gelöst. Dies erfolgt chemisch. Sobald die Verbindungsstellen gelöst sind, sorgt eine natürliche Aufdehnungstendenz des Implantats dafür, dass es seinen expandierten Zustand annimmt, in dem es im Blutgefäß deponiert werden soll. Anschließend kann der Schaft und ggf. auch der Katheter nach proximal zurückgezogen und schließlich aus dem Blutgefäßsystem entfernt werden, während das Implantat an seiner Zielposition verbleibt.

Die Begriffe "proximal" und "distal" sind so zu verstehen, dass beim Einbringen der Vorrichtung zum behandelnden Arzt weisende Teile als proximal, vom behandelnden Arzt weg weisende Teile als distal bezeichnet werden. Die Vorrichtung wird somit typischerweise durch das Blutgefäß oder einen Katheter in distaler Richtung vorgeschoben. Der Begriff "axial" bezieht sich auf die von proximal nach distal verlaufende Längsachse der Vorrichtung, der Begriff "radial" auf hierzu senkrechte Ebenen.

Um die natürliche Aufdehnungstendenz des Implantats zu gewährleisten, ist es sinnvoll, das Implantat als selbstexpandierendes Implantat vorzusehen. Bevorzugt wird das Implantat daher aus einem Formgedächtnismaterial gefertigt, wie es für die Herstellung von Stents u. ä. grundsätzlich bekannt ist. Als Materialien kommen daher insbesondere Formgedächtnislegierungen in Frage, etwa binäre Nickel-Titan-Legierungen (z. B. Nitinol) oder ternäre Nickel-Titan-Chrom- und Nickel-Titan-KupferLegierungen. Insbesondere Nitinol ist für die Anwendung in selbstexpandierenden Strukturen im medizinischen Bereich bekannt. Ebenso sind jedoch auch andere Formgedächtnismaterialien, beispielsweise andere Legierungen oder auch Formgedächtnispolymere denkbar.

Das mindestens bereichsweise, vorzugsweise insgesamt zylindrisch aufgebaute Implantat weist in der Regel über die Mantelfläche des Zylinders verteilt Öffnungen auf. Mit anderen Worten handelt es sich um eine Gitter- oder Maschenstruktur, aufgebaut aus Streben, sodass sich auf der Mantelfläche des Zylinders eine Vielzahl von Öffnungen oder Maschen ergibt.

Der Ausdruck "Öffnung" bezieht sich auf die Gitterstruktur, unabhängig von der Frage, ob die Öffnung durch eine Membran von der Umgebung abgekoppelt ist, d. h. auch eine von einer Membran abgedeckte Öffnung wird als Öffnung bezeichnet. Eine Membran kann bei Bedarf außen oder innen auf die Gitterstruktur aufgebracht sein. Ebenso möglich ist die Einbettung der Gitterstruktur in eine Membran.

Die Gitterstruktur des Implantats kann eine geflochtene Struktur sein, d. h. aus einzelnen Drähten bzw. Drahtbündeln als Streben bestehen, die miteinander verflochten sind und an den Kreuzungspunkten der Drähte/Drahtbündel über- und untereinander her verlaufen. Ebenso kann es sich um eine geschnittene Struktur handeln, bei der aus einem Rohr geeigneten Durchmessers mit Hilfe eines Lasers die Gitterstruktur herausgeschnitten wird. Das Material ist in der Regel ein Metall, kann aber auch ein Kunststoff sein. Es muss über eine hinreichende Elastizität verfügen, die eine Kontraktion auf den Durchmesser des Schafts und andererseits bei der Freisetzung die Expansion auf den gewünschten Durchmesser erlaubt. Zusätzlich ist es sinnvoll, die Gitterstruktur einer Elektropolitur zu unterziehen, um sie glatter und abgerundeter und damit weniger traumatisch werden zu lassen. Darüber hinaus sinkt die Gefahr der Anhaftung von Keimen oder sonstigen Verunreinigungen. Die Streben oder Drähte können einen runden, ovalen, quadratischen, rechteckigen oder trapezförmigen Querschnitt aufweisen, wobei im Falle eines quadratischen, rechteckigen oder trapezförmigen Querschnitts eine Abrundung der Kanten von Vorteil ist. Möglich ist auch die Verwendung von flachen Stegen/Drähten in Form dünner Streifen, insbesondere Metallstreifen.

Bei dem Implantat als Bestandteil der erfindungsgemäßen Vorrichtung kann es sich um unterschiedliche, aus dem Stand der Technik grundsätzlich bekannte Implantate handeln. Voraussetzung ist, dass das Implantat über einen inneren Hohlraum verfügt, so dass der längliche Schaft durch das Implantat hindurch verlaufen kann und das Implantat auf dem Schaft sitzt. Beispiele für solche Implantate sind Stents, Flow Diverter oder andere Implantate, die vor einem Aneurysma oder einer sonstigen vaskulären Fehlbildung platziert werden, um dadurch den Blutfluss zu beeinflussen. Hierzu gehören auch Implantate zur Platzierung im Bereich von Bifurkationsaneurysmen, wie sie beispielsweise in der WO 2014/029835 A1 beschrieben werden.

Das Implantat als Bestandteil der erfindungsgemäßen Vorrichtung hat in der Regel eine Länge zwischen 5 mm und 100 mm und einen Durchmesser zwischen 1,5 mm und 7 mm, wobei sich die Dimensionen aus der Dimension des Gefäßsegments ergeben. Die Angaben beziehen sich auf den freien, entspannten Zustand des Implantats, d. h. gelöst vom Schaft und ohne Ausübung eines äußeren Zwangs durch einen Katheter. Die das Implantat ausbildenden Streben können z. B. eine Breite bzw. einen Durchmesser zwischen 20 und 60 µm aufweisen.

Ein wesentlicher Vorteil der erfindungsgemäßen Vorrichtung ist darin zu sehen, dass das Implantat auch ohne Mikrokatheter an seinen Bestimmungsort gebracht werden kann und daher weniger Limitierungen hinsichtlich der Größe unterliegt. Darüber hinaus bleibt es bis zu seiner Ablösung proximal und distal fixiert, sodass beim Vorschub des Implantats durch ein Gefäß oder einen Katheter keine Gefahr der Stauchung, Streckung oder Knickung gegeben ist. Des Weiteren ist ein Vorteil darin zu sehen, dass im Wesentlichen nur das eigentliche Implantat mit seinen zur Erzielung der beabsichtigten Wirkung notwendigen Bestandteilen im Blutgefäß verbleibt, nicht jedoch nach Ablösung eigentlich überflüssige Verbindungselemente zum Führungsdraht oder einer sonstigen Einführvorrichtung. Dies kann bspw. wichtig sein, wenn im Blutgefäß nur ein vergleichsweise kurzer Abschnitt zur Verfügung steht, in dem das Implantat platziert werden kann, ohne abzweigende Blutgefäße zu beeinträchtigen.

Das Implantat weist proximal und distal jeweils mindestens eine Verbindungsstelle mit dem Schaft auf. Bevorzugt können sich die Verbindungsstellen jeweils am distalen und proximalen Ende des Implantats befinden, denkbar ist jedoch auch, die Verbindungsstellen zwar im proximalen und distalen Bereich des Implantats, jedoch etwas beabstandet vom jeweiligen Ende des Implantats vorzusehen. Das Implantat sollte vorzugsweise im Wesentlichen über seine gesamte Länge auf dem Schaft fixiert werden, solange keine Ablösung erfolgt.

Um zu verhindern, dass sich das Implantat teilweise vom Schaft löst, ist es von Vorteil, wenn das Implantat proximal und distal jeweils mindestens zwei Verbindungsstellen zum Schaft aufweist, bevorzugt jeweils am proximalen und distalen Ende des Implantats. Ggf. können auch mehr Verbindungsstellen vorgesehen sein, um eine weitgehende Fixierung über den radialen Umfang des Implantats am Schaft herbeizuführen. Zusätzlich können auch Verbindungsstellen zwischen proximalem und distalem Ende des Implantats vorgesehen sein. Da das Implantat in seiner auf dem Schaft festgelegten Position in einer gestreckten Konfiguration vorliegt und durch die proximal und distal vorgesehenen Verbindungsstellen in dieser Konfiguration gehalten wird, sind weitere Verbindungsstellen zwischen proximalem und distalem Ende in der Regel allerdings nicht erforderlich.

Gemäß einer alternativen Ausführungsform, für die im Übrigen das zuvor gesagte gilt, insbesondere zum Aufbau und zum Material des Implantats, wird ein System gemäß Anspruch 4 zur Verfügung gestellt.

Auch gemäß dieser alternativen Ausführungsform ist das Implantat auf der Außenseite des Schafts angebracht und wird auf diesem in seiner komprimierten Form gehalten, bis eine Ablösung erfolgt. Allerdings verbinden die Verbindungsstellen nicht den Schaft mit dem Implantat, sondern den Schaft mit einem oder mehreren Fäden, die über das Implantat verlaufen. Vorzugsweise verläuft zumindest ein Faden spiralförmig um das Implantat herum, d. h. der Faden ist spiralförmig um das Implantat gewunden und sorgt auf diese Weise dafür, dass das Implantat auf dem Schaft fixiert und in seinem komprimierten Zustand gehalten wird. Im Übrigen ist das Verfahren zum Einbringen des Implantats ähnlich der ersten Ausführungsform, d. h. der Schaft wird samt Implantat an die gewünschte Stelle gebracht. Zumeist erfolgt der Vorschub des Schafts mit dem Implantat an die Zielposition nicht durch einen Mikrokatheter sondern durch das Blutgefäß selbst. In der Regel wird allerdings wiederum wie bei der ersten Ausführungsform der Erfindung zunächst ein relativ großlumiger Führungskatheter verwendet, um den Schaft mit Implantat vorzuschieben, verzichtet wird lediglich auf den kleinlumigen Mikrokatheter für den Vorschub weiter distal. Bei neurovaskulären Anwendungen kann beispielsweise ein Vorschub durch den Führungskatheter von der Leiste bis zur Arteria carotis erfolgen, der weitere Vorschub erfolgt sodann ohne Mikrokatheter.

Möglich bleibt jedoch selbstverständlich weiterhin die Einbringung von Schaft und Implantat durch einen Katheter bis an die Zielposition. In diesem Fall werden Schaft und Katheter dort relativ zueinander in der Weise bewegt, dass das Implantat und zumindest der Teil des Schafts, der das Implantat trägt, nicht mehr innerhalb des Katheters liegen.

Sobald sich der behandelnde Arzt zur endgültigen Ablösung des Implantats entschieden hat, wird zumindest eine der Verbindungsstellen zwischen Schaft und Faden gelöst. Der Faden ist sodann nicht mehr in der Lage, das Implantat im komprimierten Zustand auf dem Schaft festzuhalten, sondern liegt nur noch lose vor. Das Implantat kann sich somit unter Ausnutzung seiner natürlichen Aufdehnungstendenz zu seiner expandierten Form aufweiten und sich im Blutgefäß festsetzen. Der nunmehr nur noch locker durch das Innere des Implantats verlaufende Schaft kann sodann problemlos in Richtung proximal zurückgezogen und aus dem Blutgefäßsystem entfernt werden.

Bevorzugt ist bei der zweiten Ausführungsform insbesondere die distale Verbindungsstelle bzw. sind im Falle mehrerer Fäden die distalen Verbindungsstellen zwischen Faden und Schaft lösbar ausgebildet. Die proximale Verbindungsstelle zwischen Faden und Schaft bleibt hingegen bevorzugt erhalten. Die distale Ablösung des Fadens ist mit dem Vorteil verbunden, dass beim Zurückziehen des Schafts nach Freisetzung des Implantats der Faden proximal mit dem Schaft verbunden bleibt und mit zurückgezogen wird, ohne dass der Faden umschlägt. Ein Umschlagen des Fadens wäre mit dem Risiko behaftet, dass der Faden die Gefäßwand verletzt oder am Implantat hängenbleibt.

Vorzugsweise befinden sich die Verbindungsstellen zwischen Schaft und Faden jeweils am proximalen und distalen Ende des Fadens, damit über die Verbindungsstelle hinaus keine Bereiche des Fadens nach proximal oder distal überstehen. Denkbar ist jedoch auch ein Vorsehen der Verbindungsstellen mit einem gewissen Abstand vom proximalen bzw. distalen Ende des Fadens, solange gewährleistet ist, dass der Faden das Implantat in seinem komprimierten Zustand sicher auf dem Schaft fixiert. Sinnvollerweise erstreckt sich der Faden über die gesamte Länge des Implantats, vorzugsweise spiralförmig um das Implantat herum.

Sowohl bei der ersten als auch bei der zweiten Ausführungsform der Erfindung handelt es sich bei den Verbindungsstellen um chemische Verbindungsstellen, d. h. die Verbindungsstellen sind durch chemische Einwirkung, nämlich Applikation eines Lösungsmittels, zumindest so weitgehend auflösbar, dass sich das Implantat bzw. der Faden vom Schaft löst.

Bei den Verbindungsstellen handelt es sich um Klebeverbindungsstellen, wobei bevorzugt ein Polymerklebstoff verwendet wird. Die Ablösung des Implantats bzw. Fadens erfolgt in diesem Fall chemisch, nämlich durch Applikation eines den Klebstoff zumindest teilweise auflösenden Lösungsmittels. Als Lösungsmittel kann z. B. DMSO (Dimethylsulfoxid) verwendet werden.

Im Falle der Klebeverbindungsstellen wird das Lösungsmittel durch das Innere des Schafts zu den Verbindungsstellen gebracht. Dies kann durch einen Schlauch oder auch durch einen hierfür im Inneren des Schafts vorgesehenen Tubus erfolgen. Voraussetzung hierfür ist selbstverständlich, dass der Schaft zum einen einen inneren Hohlraum aufweist und zum anderen eine gewisse Durchlässigkeit der Außenwandung des Schafts, damit das innen applizierte Lösungsmittel durch den Schaft hindurchtreten und die Klebeverbindungsstelle lösen kann. Schließlich ist es auch möglich, ein oder mehrere Lösungsmittelreservoire im Bereich der Verbindungsstellen vorzusehen, die sich von extern so steuern und öffnen lassen, dass eine Einwirkung des Lösungsmittels auf die Verbindungsstelle erfolgt. Derartige Lösungsmittelreservoire können prinzipiell auch außen auf dem Schaft vorgesehen sein, sofern eine Steuerung zur Freisetzung des Lösungsmittels von außen gegeben ist.

Unabhängig von der genauen Ausgestaltung der Verbindungsstellen weist der Schaft einen in Längsrichtung des Schafts verlaufenden inneren Hohlraum auf, mit anderen Worten ein inneres Lumen. Zum einen dient dies, wie bereits ausgeführt, der Lösung der Verbindungsstellen, zum anderen ist ein Lumen auch insofern vorteilhaft, als sich durch das Lumen ein Führungsdraht erstrecken kann. Entsprechend kann zunächst ein Führungsdraht bis zur gewünschten Stelle vorgeschoben werden, um anschließend den Schaft mit Implantat über den Führungsdraht zu schieben. Nach erfolgter Positionierung des Schafts kann sodann der Führungsdraht wieder nach proximal zurückgezogen werden. Das Lumen des Schafts muss einen Durchmesser aufweisen, der ein Vorschieben des Schafts über den Führungsdraht erlaubt. Der innere Hohlraum des Schafts kann sich vom proximalen bis zum distalen Ende des Schafts erstrecken. Entsprechend betrifft die Erfindung auch eine Kombination der erfindungsgemäßen Vorrichtung mit einem Führungsdraht.

Wenn sich der innere Hohlraum des Schafts vom proximalen bis zum distalen Ende erstreckt und auch der Führungsdraht durch den gesamten Schaft verläuft, spricht man von einem Over-The-Wire (OTW)-System. Alternativ ist auch ein Rapid Exchange (Rx)-System denkbar. Hierbei verläuft der Führungsdraht nur durch den distalen Abschnitt des Schafts, wobei der distale Abschnitt typischerweise eine Länge von ca. 20 bis 40 cm hat. Entsprechend weist der Schaft proximal des distalen Abschnitts eine Durchtrittsöffnung auf, durch die der Führungsdraht verläuft und das Lumen des Schafts verlässt. Eine solche Durchtrittsöffnung wird auch als Port bezeichnet. OTW- und Rx-System sind beispielsweise im Bereich von Ballonkathetern dem Fachmann geläufig. Auch bei Vorliegen einer Durchtrittsöffnung kann der Schaft einen sich von proximal nach distal erstreckenden inneren Hohlraum aufweisen, der in diesem Fall in erster Linie der Einbringung oder Zufuhr von Mitteln zur chemischen Lösung der Verbindungsstellen dient, wie zuvor beschrieben.

Der Schaft ist zumindest in den Abschnitten, an denen sich die zu lösenden Klebeverbindungsstellen befinden, für das Lösungsmittel durchlässig, damit das Lösungsmittel durch die Wandung des Schafts nach außen dringen und die Klebeverbindungsstelle lösen kann. Die Erfindung betrifft somit auch eine Vorrichtung, die ein Zufuhrsystem für Lösungsmittel aufweist, wobei das Zufuhrsystem zumindest temporär durch den inneren Hohlraum des Schafts verläuft, so dass ein Lösungsmittel an die Stellen auf der Innenseite des Schafts bringbar ist, die den Stellen auf der Außenseite entsprechen, an denen der Schaft Klebeverbindungsstellen aufweist, die zur Freisetzung des Implantats gelöst werden müssen. Das Zufuhrsystem kann unterschiedlich ausgestaltet sein; es kann sich um Schläuche oder Tuben handeln, die durch das innere Lumen des Schafts verlaufen, sodass das Lösungsmittel von proximal nach distal durch den gesamten Schaft oder einen Teil des Schafts geführt wird. Alternativ muss kein gesonderter Tubus vorgesehen werden, sondern das Lösungsmittel wird durch das Lumen des Schafts geführt.

Gemäß einer bevorzugten Ausführungsform ist der Schaft zumindest teilweise aus einer Wendel aufgebaut, die sich aus dem helixförmigen Verlauf eines Metalldrahtes oder eines Kunststofffadens ergibt. Dies betrifft insbesondere den distalen Bereich des Schafts bzw. den Bereich, der das Implantat trägt. Der wendelförmige Aufbau sorgt zum einen für eine erhöhte Flexibilität des Schafts, was gerade beim Durchführen eines Schafts durch englumige, kurvenreiche Blutgefäße mit kleinen Kurvenradien von Vorteil ist, wie sie beispielsweise neurovaskulär vorkommen. Darüber hinaus können die Zwischenräume zwischen den einzelnen Windungen der Wendel/Coil so ausgebildet sein, dass sie lösungsmitteldurchlässig sind.

Entsprechend kann bei chemischer Lösung der Verbindungsstellen zwischen Schaft und Implantat bzw. Schaft und Faden Lösungsmittel im Inneren des Schafts freigesetzt werden, durch die Zwischenräume zwischen den Windungen treten und die Verbindungsstelle lösen. Zusätzliche Ausnehmungen im Schaft sind somit entbehrlich.

Für die Wendel (Coil) können verschiedene Materialien verwendet werden, bspw. typische Metalle, wie sie für endovaskuläre Vorrichtungen zum Einsatz kommen. Beispielhaft seien Edelstahl, Kobalt-Chrom-Legierungen und Nickel-Titan-Legierungen genannt. Möglich ist auch die Verwendung von medizinisch kompatiblen Kunststoffen.

Eine Alternative zur Verwendung einer Wendel im distalen Bereich des Schafts ist die Verwendung eines Schlauchmaterials, das ebenfalls über eine ausreichende Flexibilität verfügen sollte. Je nachdem, welche Form der Lösbarkeit für die Verbindungsstellen gewählt wird, können im Schlauchmaterial zusätzliche Ausnehmungen vorhanden sein, die es erlauben, ein Lösungsmittel oder auch einen elektrischen Kontakt an die zu lösenden Verbindungsstellen zu bringen.

Während es für den distalen Bereich des Schafts vorteilhaft ist, wenn eine hinreichende Flexibilität gegeben ist, kann der weiter proximal liegende Bereich des Schafts eine größere Steifigkeit aufweisen. Da der Schaft innerhalb des Blutgefäßsystems häufig über vergleichweise große Distanzen vorgeschoben werden muss, ist es sinnvoll, den proximalen Bereich steifer auszubilden, während im distalen Bereich, der beispielsweise bei neurovaskulären Anwendungen in fein verästelte, englumige Blutgefäße vordringen muss, eine höhere Flexibilität von Vorteil ist.

Das proximale Ende des Schafts kann beispielsweise in einem üblichen Anschluss (Hub) bestehen. Zweckmäßig ist die Verwendung eines üblichen Luer- oder Luer-Lock-Anschlusses. Über einen solchen Anschluss können Hilfsmittel in das Innere des Schafts eingeführt werden, beispielsweise ein Führungsdraht oder Mittel zur Herbeiführung einer Ablösung der Verbindungsstellen. Dies können Mittel zur Zufuhr eines Lösungsmittels, zur Herbeiführung einer Erwärmung in einem Bereich des Schafts oder zum Anlegen einer elektrischen Spannung sein. Zumeist wird das innere Lumen des Anschlusses in Richtung distal, d. h. zum inneren Lumen des Schafts selbst hin enger. Im Falle eines Luer-Lock-Anschlusses erfolgt der Anschluss an weitere Elemente zusätzlich durch Verschraubung mit dem hierfür vorgesehenen Gewinde. Das Anschlusselement kann z. B. aus Polycarbonat, Polyamid, Polypropylen oder anderen Polymeren hergestellt sein. In der Regel ist das Material steifer als das Material für den eigentlichen Schaft.

Die Erfindung lässt sich insbesondere im neurovaskulären Bereich anwenden, ebenso möglich sind jedoch Anwendungen der Vorrichtungen in anderen Bereichen des Blutgefäßsystems, beispielsweise kardiovaskulär und peripher.

Sinnvollerweise verfügt die Vorrichtung über ein oder mehrere röntgendichte Markierungen, um dem behandelnden Arzt eine Visualisierung zu ermöglichen. Die röntgendichten Markierungen können z. B. aus Platin, Palladium, Platin-Iridium, Tantal, Gold, Wolfram oder anderen röntgendichten Metallen sein. Sie erlauben es dem behandelnden Arzt zu erkennen, ob die Vorrichtung, insbesondere das auf dem Schaft befindliche Implantat, korrekt platziert ist, und ggf. Korrekturen vorzunehmen. Denkbar ist auch, zumindest einige Bereiche des Schafts und/oder des Implantats mit einer Beschichtung aus einem röntgendichten Material zu versehen, beispielsweise mit einer Goldbeschichtung. Diese kann z. B. eine Stärke von 1 bis 6 µm aufweisen. Auch beim Vorsehen einer röntgendichten Beschichtung kann es allerdings sinnvoll sein, zusätzlich einen oder mehrere röntgendichte Markierungen anzubringen. Eine weitere Möglichkeit besteht darin, einzelne Streben des Implantats mit einer Helix oder einem Draht aus einem röntgendichten Material wie Platin zu ummanteln.

Neben der erfindungsgemäßen Vorrichtung betrifft die Erfindung auch ein Verfahren zur Einbringung von Implantaten in Blutgefäße. Bei diesem Verfahren wird das Implantat auf dem Schaft festgelegt und in das Blutgefäß eingeführt. Anschließend löst man bei der ersten Ausführungsformen sämtliche Verbindungsstellen zwischen Implantat und Schaft, um das Implantat freizusetzen. Das Implantat expandiert zu der Form, die das Implantat im freigesetzten Zustand aufweist und setzt sich im Blutgefäß fest. Der Schaft selbst ist nicht mehr mit dem Implantat verbunden und kann somit nach proximal zurückgezogen und aus dem Blutgefäßsystem entfernt werden.

Bei der zweiten Ausführungsform, bei der das Implantat über einen Faden am Schaft gehalten wird, geht man grundsätzlich ähnlich vor, allerdings wird hier in der Regel nur eine Verbindungsstelle zwischen Faden und Schaft gelöst, so dass der Faden das Implantat nicht mehr im komprimierten Zustand am Schaft hält. Unter Umständen können auch mehrere Verbindungsstellen zwischen einem oder mehreren Fäden und dem Schaft gelöst werden; wichtig ist, dass der oder die Fäden nach dem Lösen nicht mehr für eine Komprimierung des Implantats und Festlegung am Schaft sorgen, sondern sich das Implantat entfalten kann. Der Schaft weist somit keine feste Verbindung mehr mit dem Implantat auf und kann proximal aus dem Blutgefäßsystem entfernt werden. Sämtliche Ausführungen, die im Zusammenhang mit den Vorrichtungen gemacht wurden, gelten in gleicher Weise auch für das hier beschriebene Verfahren und umgekehrt.

Die Erfindung wird anhand der in den Figuren veranschaulichten Ausführungsbeispiele beispielhaft näher erläutert. Es ist darauf hinzuweisen, dass die Figuren bevorzugte Ausführungsvarianten der Erfindung zeigen, die Erfindung ist jedoch nicht hierauf beschränkt. Allgemein umfasst die Erfindung, soweit es technisch sinnvoll ist, beliebige Kombinationen der technischen Merkmale, die in den Ansprüchen aufgeführt oder in der Beschreibung beschrieben sind. Sämtliche Ausführungen, die zur ersten Ausführungsform der Erfindung mit Verbindungsstellen zwischen Schaft und Implantat gemacht sind, gelten in gleicher Weise für die zweite Ausführungsform mit Verbindungsstellen zwischen Schaft und Faden und umgekehrt, soweit sich nicht aus dem Zusammenhang etwas anderes ergibt.

Es zeigen:
- Fig. 1: den Schaft der erfindungsgemäßen Vorrichtung in der Seitenansicht;
- Fig. 2: die erfindungsgemäße Vorrichtung gemäß einer ersten Ausführungsform in der Seitenansicht mit am Schaft festgelegtem Implantat;
- Fig. 3: die erfindungsgemäße Vorrichtung gemäß einer ersten Ausführungsform in der Seitenansicht mit vom Schaft gelöstem Implantat;
- Fig. 4: die erfindungsgemäße Vorrichtung gemäß einer zweiten Ausführungsform in der Seitenansicht mit am Schaft festgelegtem Implantat;
- Fig. 5: die erfindungsgemäße Vorrichtung gemäß einer zweiten Ausführungsform in der Seitenansicht mit vom Schaft gelöstem Implantat und
- Fig. 6: das freigesetzte Implantat.

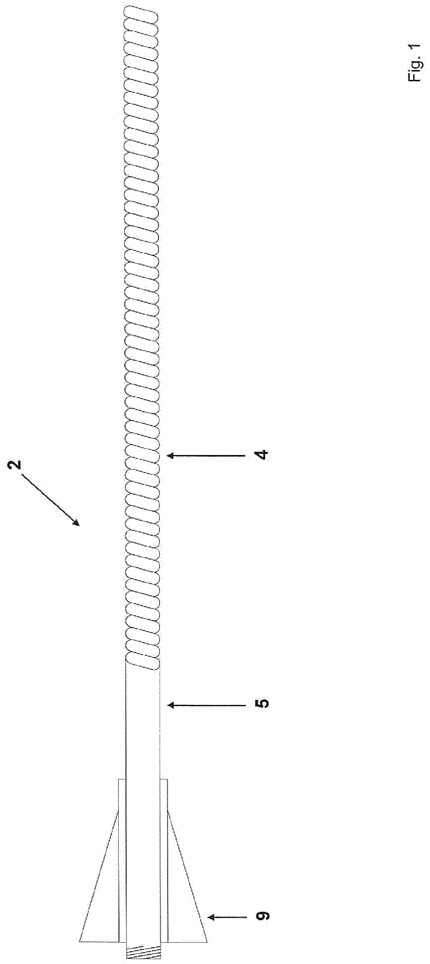

In Figur 1 wird ein Schaft 2, wie er Bestandteil der erfindungsgemäßen Vorrichtung ist, in der Seitenansicht gezeigt, wobei in der gewählten Darstellung links proximal und rechts distal bedeutet. Der Schaft 2 weist einen proximalen Bereich 5 mit vergleichsweise großer Steifigkeit sowie eine flexible Wendel 4 als distalen Bereich des Schafts 2 auf. Das proximale Ende des Schafts 2 bildet das Anschlusselement 9 in Form eines Luer-Lock-Anschlusses mit Gewinde.

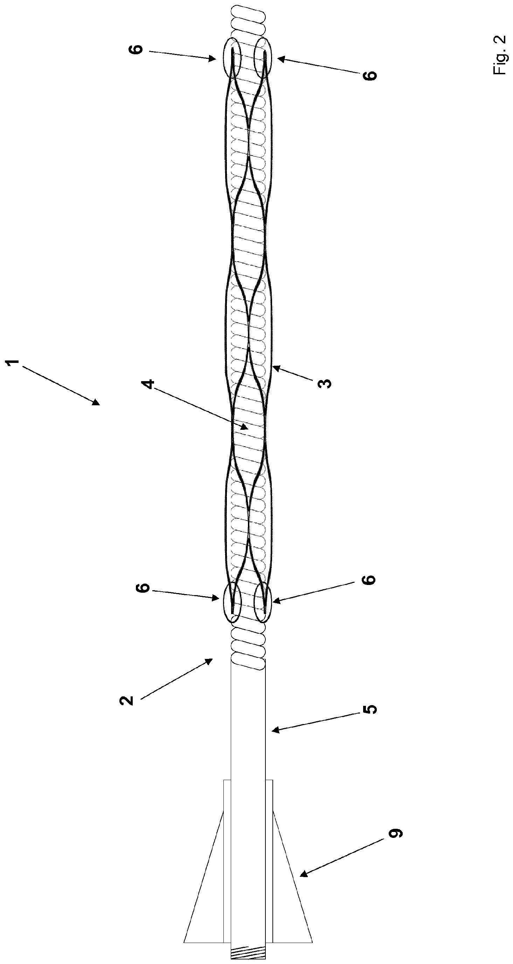

In Figur 2 ist die Vorrichtung 1 gemäß einer ersten Ausführungsform gezeigt. Der Schaft 2 trägt hier das Implantat 3, bei dem es sich um einen lasergeschnittenen Stent handelt. Das Implantat 3 ist über mehrere Verbindungsstellen 6 mit der Wendel 4 des Schafts 2 verbunden. Bei den Verbindungsstellen 6 handelt es sich hier um Klebeverbindungsstellen, die durch Applikation eines Lösungsmittels lösbar sind. Das Lösungsmittel kann durch das Innere des Schafts 2 an die Verbindungsstellen 6 gebracht werden, da die Zwischenräume zwischen den Windungen der Wendel 4 ausreichend durchlässig sind.

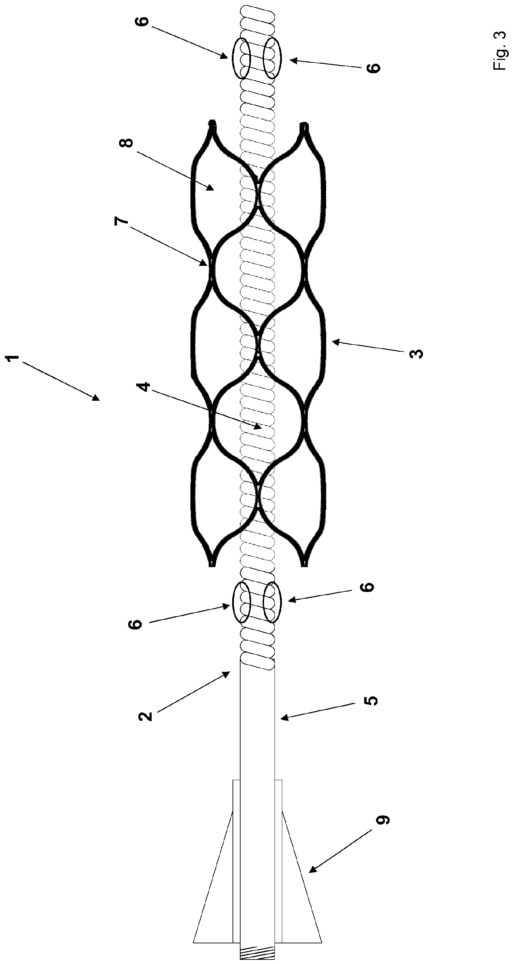

Wenn die Verbindungsstellen 6 gelöst wurden, besteht keine unmittelbare Verbindung mehr zwischen Implantat 3 und Wendel 4, wie in Figur 3 dargestellt. Da das Implantat 3 selbstexpandierend ist, weitet es sich aus und nimmt im Blutgefäß seine endgültige Position ein. Der Schaft 2 bzw. der distale Bereich des Schafts, d. h. die Wendel 4 verläuft nur noch lose durch das Innere des Implantats 3 und kann problemlos in Richtung proximal zurückgezogen werden. Das Implantat 3 weist Streben 7 auf, zwischen denen Öffnungen 8 ausgebildet sind.

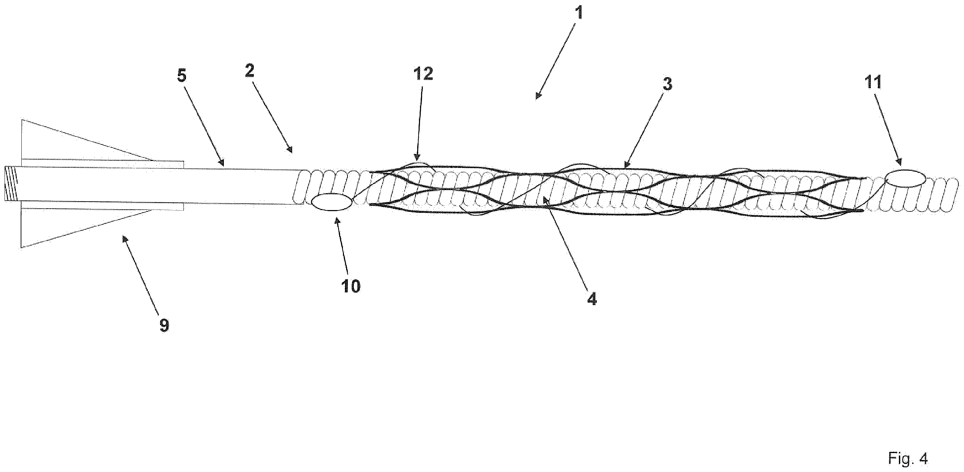

In Figur 4 ist die zweite Ausführungsform beispielhaft dargestellt. Bei dieser befindet sich das Implantat 3 ebenfalls auf der den distalen Bereich des Schafts 2 ausbildenden Wendel 4, im Gegensatz zur ersten Ausführungsform verbinden die Verbindungsstellen jedoch nicht unmittelbar das Implantat 3 mit der Wendel 4, vielmehr wird über Verbindungsstellen 10, 11 ein Faden 12 fixiert. Der Faden 12 windet sich um das Implantat 3 herum und hält dieses somit in seiner komprimierten Form auf der Wendel 4 des Schafts 2. Das Implantat 3 hat eine natürliche Aufweitungstendenz, wird jedoch so lange an der Aufweitung gehindert, wie der Faden 12 um das Implantat 3 gewunden ist.

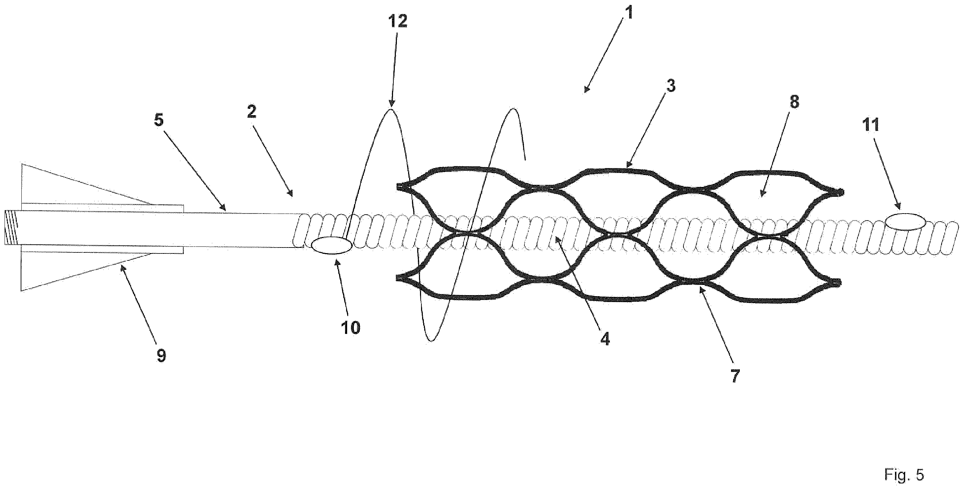

In Figur 5 wird die Freisetzung gemäß der zweiten Ausführungsform dargestellt. Die distale Verbindungsstelle 11 wird gelöst, d. h. am distalen Ende hat der Faden 12 keine Verbindung mehr mit der Wendel 4. Die Lösung der Verbindungsstelle 11 erfolgt wiederum in der Weise, dass ein Lösungsmittel auf die als Klebeverbindungsstelle ausgebildete Verbindungsstelle 11 einwirkt, wobei das Lösungsmittel vom Inneren der Wendel her durch die Zwischenräume zwischen den Windungen der Wendel 4 durchdringt. Im Gegensatz dazu bleibt die Verbindungsstelle 10 am proximalen Ende des Fadens 12 erhalten.

Da der Faden 12 nicht mehr fest um das Implantat 3 gewunden ist, kann sich das Implantat 3 aufgrund seiner Formgedächtniseigenschaften aufweiten und setzt sich im Blutgefäß fest. Die Wendel 4 des Schafts 2 verläuft nur noch ohne Verbindung zum Implantat 3 durch das Lumen des Implantats 3 und kann in Richtung proximal zurückgezogen und aus dem Blutgefäßsystem entfernt werden. Zugleich wird der Faden 12, der weiterhin an der proximalen Verbindungstelle 10 über eine feste Verbindung mit der Wendel 4 verfügt, mit in Richtung proximal zurückgezogen und ebenfalls entfernt.

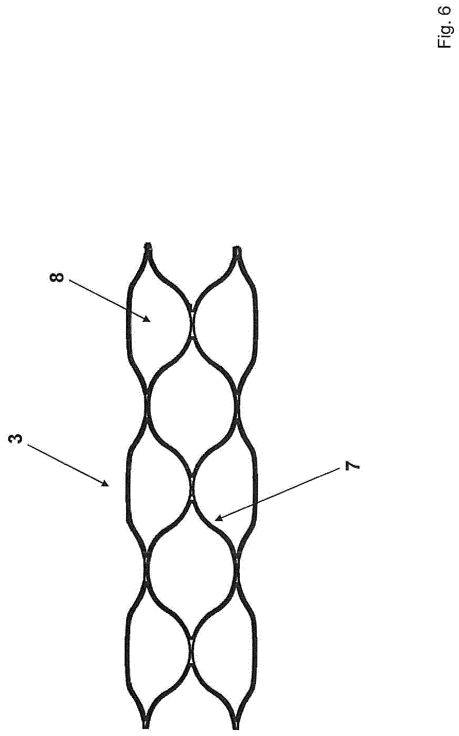

In Figur 6 schließlich ist das freigesetzte Implantat 3 gezeigt, dass in dieser Form im Blutgefäß implantiert wird. Beim Implantat 3 handelt es sich um einen geschnittenen Stent, dessen Streben 7 diverse Öffnungen 8 zwischen sich ausbilden.

## Patentansprüche

1. System aus einem endovaskulären, zumindest teilweise zylindrisch aufgebauten Implantat (3) und einer Vorrichtung (1) zur Einbringung des Implantats (3) in Blutgefäße, wobei das Implantat (3) in einem expandierten Zustand, in dem es im Blutgefäß platziert wird, und in einem komprimierten Zustand vorliegt, in dem es in das Blutgefäß einführbar ist, wobei die Vorrichtung (1) einen länglichen Schaft (2) aufweist, auf dessen Außenseite das Implantat (3) im komprimierten Zustand ablösbar festgelegt ist und der durch das Implantat (3) hindurch verläuft, wobei das Implantat (3) proximal und distal jeweils mindestens eine Verbindungsstelle (6) mit dem Schaft (2) aufweist,
**dadurch gekennzeichnet,**
**dass** die Verbindungsstellen (6) chemisch lösbare Klebeverbindungsstellen sind, der längliche Schaft (2) einen in Längsrichtung des Schafts (2) verlaufenden inneren Hohlraum aufweist und der Schaft (2) zumindest in den Abschnitten, an denen sich Klebeverbindungsstellen befinden, durchlässig für ein Lösungsmittel ist, das in der Lage ist, die Klebeverbindungsstellen aufzulösen und eine Ablösung des Implantats (3) vom Schaft (2) zu bewirken.

2. System nach Anspruch 1, **dadurch gekennzeichnet, dass** sich zumindest eine Verbindungsstelle (6) am proximalen Ende des Implantats (3) und zumindest eine Verbindungsstelle (6) am distalen Ende des Implantats (3) befindet.

3. System nach Anspruch 1 oder 2, **dadurch gekennzeichnet, dass** das Implantat (3) proximal und distal jeweils mindestens zwei Verbindungsstellen (6) mit dem Schaft (2) aufweist.

4. System aus einem endovaskulären, zumindest teilweise zylindrisch aufgebauten Implantat (3) und einer Vorrichtung (1) zur Einbringung des Implantats (3) in Blutgefäße, wobei das Implantat (3) in einem expandierten Zustand, in dem es im Blutgefäß platziert wird, und in einem komprimierten Zustand vorliegt, in dem es in das Blutgefäß einführbar ist, wobei die Vorrichtung (1) einen länglichen Schaft (2) aufweist, auf dessen Außenseite das Implantat (3) im komprimierten Zustand ablösbar festgelegt ist und der durch das Implantat (3) hindurch verläuft, **dadurch gekennzeichnet,**
**dass** sich zumindest ein Faden (12) in Längsrichtung über das Implantat (3) in der Weise zumindest über einen Großteil der Länge des Implantats (3) erstreckt, dass das Implantat (3) auf dem Schaft (2) im komprimierten Zustand festgelegt wird, wobei der Faden (12) proximal und distal jeweils mindestens eine Verbindungsstelle (10, 11) mit dem Schaft (2) aufweist, wobei die Verbindungsstellen (10, 11) chemisch lösbare Klebeverbindungsstellen sind, wobei der längliche Schaft (2) einen in Längsrichtung des Schafts (2) verlaufenden inneren Hohlraum aufweist und der Schaft (2) zumindest in den Abschnitten, an denen sich Klebeverbindungsstellen befinden, durchlässig für ein Lösungsmittel ist, das in der Lage ist, die Klebeverbindungsstellen aufzulösen und eine Ablösung des Fadens (12) vom Schaft (2) zu bewirken.

5. System nach Anspruch 4, **dadurch gekennzeichnet, dass** sich der zumindest eine Faden (12) spiralförmig in Längsrichtung um das Implantat (3) erstreckt.

6. System nach einem der Ansprüche 1 bis 5, **dadurch gekennzeichnet, dass** die Klebeverbindungsstellen einen Polymerklebstoff aufweisen.

7. System nach einem der Ansprüche 1 bis 6, **dadurch gekennzeichnet, dass** sich der innere Hohlraum vom proximalen bis zum distalen Ende des Schafts (2) erstreckt.

8. System nach einem der Ansprüche 1 bis 7, **dadurch gekennzeichnet, dass** proximal eines distalen Abschnitts des Schafts (2) eine Durchtrittsöffnung für einen Führungsdraht vom inneren Hohlraum nach außen angeordnet ist.

9. System nach einem der Ansprüche 1 bis 8, **gekennzeichnet durch** ein Zufuhrsystem für Lösungsmittel, welches zumindest temporär durch den inneren Hohlraum des Schafts (2) verläuft und durch welches ein Lösungsmittel an die Stellen des Schafts (2) bringbar ist, an denen sich auf der Außenseite des Schafts (2) Klebeverbindungsstellen befinden.

10. System nach einem der Ansprüche 1 bis 9, **dadurch gekennzeichnet, dass** der Schaft (2) zumindest teilweise, insbesondere im distalen Bereich, aus einer Wendel (4) aufgebaut ist, die sich aus dem helixförmigen Verlauf eines Metalldrahts oder eines Kunststofffadens ergibt, wobei die Zwischenräume zwischen den Windungen der Wendel (4) für ein Lösungsmittel durchlässig sind.

11. System nach Anspruch 10, **dadurch gekennzeichnet, dass** sich an die den distalen Bereich des Schafts (2) ausbildende Wendel (4) proximal ein Bereich (5) des Schafts (2) anschließt, der über eine größere Steifigkeit als die Wendel (4) verfügt.

## Claims

1. System composed of an endovascular, at least partially cylindrical implant (3) and a device (1) for introducing the implant (3) into blood vessels, wherein the implant (3) is provided in an expanded state, in which the implant is placed in the blood vessel, and in a compressed state, in which the implant can be introduced into the blood vessel, wherein the device (1) has an elongated shaft (2), the exterior of which is releasably secured to the implant (3) in the compressed state and through which the implant (3) runs, wherein the implant (3) has at least one respective connection point (6) to the shaft (2) proximally and distally,
**characterised in that**
the connection points (6) are chemically releasable adhesive connection points, the elongated shaft (2) has an inner hollow space running in the longitudinal direction of the shaft (2), and the shaft (2), at least in portions where adhesive connection points are located, is permeable to a solvent capable of dissolving the adhesive connection points and causing the implant (3) to detach from the shaft (2).

2. System according to claim 1, **characterised in that** at least one connection point (6) is located at the proximal end of the implant (3) and at least one connection point (6) is located at the distal end of the implant (3).

3. System according to claim 1 or 2, **characterised in that** the implant (3) has at least two respective connection points (6) to the shaft (2) proximally and distally.

4. System composed of an endovascular, at least partially cylindrical implant (3) and a device (1) for introducing the implant (3) into blood vessels, wherein the implant (3) is provided in an expanded state, in which the implant is placed in the blood vessel, and in a compressed state, in which the implant can be introduced into the blood vessel, wherein the device (1) has an elongated shaft (2), the exterior of which is releasably secured to the implant (3) in the compressed state and through which the implant (3) runs,
**characterised in that**
at least one thread (12) extends longitudinally over the implant (3) in such a way, at least over the main part of the length of the implant (3), that the implant (3) is fixed on the shaft (2) in the compressed state, wherein the thread (12) has at least one respective connection point (10, 11) to the shaft (2) proximally and distally, wherein the connection points (10, 11) are chemically releasable adhesive connection points, wherein the elongated shaft (2) has an inner hollow space running in the longitudinal direction of the shaft (2), and the shaft (2), at least in portions where adhesive connection points are located, is permeable to a solvent capable of dissolving the adhesive connection points and causing the thread (12) to detach from the shaft (2).

5. System according to claim 4, **characterised in that** the at least one thread (12) extends spirally in the longitudinal direction around the implant (3).

6. System according to any one of claims 1 to 5, **characterised in that** the adhesive connection points have a polymer adhesive.

7. System according to any one of claims 1 to 6, **characterised in that** the inner hollow space extends from the proximal end to the distal end of the shaft (2).

8. System according to any one of claims 1 to 7, **characterised in that** proximally to a distal portion of the shaft (2) a through-opening for a guide wire is arranged from the inner hollow space to the outside.

9. System according to any one of claims 1 to 8, **characterised by** a delivery system for solvents, which runs at least temporarily through the inner hollow space of the shaft (2) and through which a solvent can be applied to those points of the shaft (2) at which adhesive connection points are arranged on the outside of the shaft (2).

10. System according to any one of claims 1 to 9, **characterised in that** the shaft (2) is composed at least partially, in particular in the distal region, of a coil (4) formed from the helical configuration of a metal wire or a plastic thread, wherein the interspaces between the windings of the coil (4) are permeable to a solvent.

11. System according to claim 10, **characterised in that** the coil (4) forming the distal region of the shaft (2) is followed proximally by a region (5) of the shaft (2) having a greater stiffness than the coil (4).

## Revendications

1. Système constitué d'un implant (3) endovasculaire de construction au moins en partie cylindrique et d'un dispositif (1) destiné à introduire l'implant (3) dans des vaisseaux sanguins, l'implant (3) se présentant dans un état déployé, dans lequel il est placé dans le vaisseau sanguin, et dans un état comprimé, dans lequel il peut être inséré dans le vaisseau sanguin, le dispositif (1) présentant une tige (2) allongée, sur la face extérieure de laquelle l'implant (3) est fixé de manière libérable dans l'état comprimé et qui s'étend à travers l'implant (3), l'implant (3) présentant côté proximal et côté distal respectivement au moins un point de liaison (6) avec la tige (2),
**caractérisé en ce que**
les points de liaison (6) sont des points de liaison collée libérables chimiquement, la tige (2) allongée présente une cavité intérieure s'étendant dans la direction longitudinale de la tige (2) et la tige (2) est perméable à un solvant au moins dans les parties sur lesquelles se trouvent des points de liaison collée, ledit solvant étant apte à dissoudre les points de liaison collée et à provoquer un détachement de l'implant (3) par rapport à la tige (2).

2. Système selon la revendication 1, **caractérisé en ce qu'**au moins un point de liaison (6) se trouve à l'extrémité proximale de l'implant (3) et au moins un point de liaison (6) se trouve à l'extrémité distale de l'implant (3).

3. Système selon la revendication 1 ou 2, **caractérisé en ce que** l'implant (3) présente, côté proximal et côté distal, respectivement au moins deux points de liaison (6) avec la tige (2).

4. Système constitué d'un implant (3) endovasculaire de construction au moins en partie cylindrique et d'un dispositif (1) destiné à introduire l'implant (3) dans des vaisseaux sanguins, l'implant (3) se présentant dans un état déployé, dans lequel il est placé dans le vaisseau sanguin, et dans un état comprimé, dans lequel il peut être inséré dans le vaisseau sanguin, le dispositif (1) présentant une tige (2) allongée, sur la face extérieure de laquelle l'implant (3) est fixé de manière libérable dans l'état comprimé et qui s'étend à travers l'implant (3),
**caractérisé en ce que**
au moins un fil (12) s'étend dans la direction longitudinale par-dessus l'implant (3) au moins sur une grande partie de la longueur de l'implant (3) de telle manière que l'implant (3) est fixé à la tige (2) dans l'état comprimé, le fil (12) présentant, côté proximal et côté distal, respectivement au moins un point de liaison (10, 11) avec la tige (2), les points de liaison (10, 11) étant des points de liaison collée libérables chimiquement, la tige (2) allongée présentant une cavité intérieure s'étendant dans la direction longitudinale de la tige (2) et la tige (2) étant perméable à un solvant au moins dans les parties sur lesquelles se trouvent des points de liaison collée, ledit solvant étant apte à dissoudre les points de liaison collée et à provoquer un détachement du fil (12) par rapport à la tige (2).

5. Système selon la revendication 4, **caractérisé en ce que** l'au moins un fil (12) s'étend en forme de spirale dans la direction longitudinale autour de l'implant (3).

6. Système selon l'une des revendications 1 à 5, **caractérisé en ce que** les points de liaison collée présentent une colle polymère.

7. Système selon l'une des revendications 1 à 6, **caractérisé en ce que** la cavité intérieure s'étend de l'extrémité proximale à l'extrémité distale de la tige (2).

8. Système selon l'une des revendications 1 à 7, **caractérisé en ce qu'**une ouverture de passage pour un fil de guidage, allant de la cavité intérieure jusqu'à l'extérieur, est disposée du côté proximal d'une partie distale de la tige (2).

9. Système selon l'une des revendications 1 à 8, **caractérisé par** un système d'arrivée de solvant, qui s'étend au moins temporairement à travers la cavité intérieure de la tige (2) et par lequel un solvant peut être amené aux points de la tige (2) au niveau desquels se trouvent des points de liaison collée sur la face extérieure de la tige.

10. Système selon l'une des revendications 1 à 9, **caractérisé en ce que** la tige (2) est construite au moins en partie, en particulier dans la zone distale, à partir d'une hélice (4), qui résulte du parcours hélicoïdal d'un fil métallique ou d'un fil en matière plastique, les espaces intermédiaires entre les spires de l'hélice (4) étant perméables à un solvant.

11. Système selon la revendication 10, **caractérisé en ce qu'**une zone (5) de la tige (2) qui dispose d'une rigidité supérieure à celle de l'hélice (4) est dans le prolongement, côté proximal, de l'hélice (4) formant la zone distale de la tige (2).
